(19) Europäisches Patentamt
European Patent Office
Office européen des brevets

(11) Veröffentlichungsnummer: **0 349 856 A2**

(12) **EUROPÄISCHE PATENTANMELDUNG**

(21) Anmeldenummer: 89111517.2

(51) Int. Cl.4: C07D 411/04 , C07D 249/08

(22) Anmeldetag: 24.06.89

(30) Priorität: 08.07.88 DE 3823174

(43) Veröffentlichungstag der Anmeldung: 10.01.90 Patentblatt 90/02

(84) Benannte Vertragsstaaten: BE CH DE FR GB IT LI

(71) Anmelder: BAYER AG
D-5090 Leverkusen 1 Bayerwerk(DE)

(72) Erfinder: Kaulen, Johannes, Dr.
Leuchter Gemark 2
D-5060 Bergisch Gladbach 2(DE)

# (54) Optisch aktive Oxirane.

(57) Neue optisch aktive Oxirane der Formel

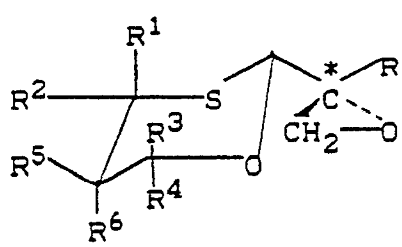

(I)

in welcher
R für gegebenenfalls substituiertes Alkyl, gegebenenfalls substituiertes Cycloalkyl oder für gegebenenfalls substituiertes Phenyl steht und
$R^1$, $R^2$, $R^3$, $R^4$, $R^5$ und $R^6$ für Wasserstoff oder Alkyl stehen, wobei jedoch mindestens einer der Reste für Alkyl steht, oder
$R^4$ und $R^5$ gemeinsam für gegebenenfalls durch Alkyl substituiertes Alkandiyl stehen oder
$R^4$ und $R^5$ gemeinsam mit den angrenzenden Kohlenstoffatomen für einen gegebenenfalls durch Alkyl substituierten, anellierten bicyclischen Kohlenwasserstoff-Rest stehen,
ein neues Verfahren zur Herstellung der erfindungsgemäßen Stoffe und ihre Verwendung als Zwischenprodukte zur Synthese von optisch aktiven Wirkstoffen mit fungiziden und pflanzenwuchsregulierenden Eigenschaften.

EP 0 349 856 A2

## Optisch aktive Oxirane

Die vorliegende Erfindung betrifft neue optisch aktive Oxirane, ein neues Verfahren zu ihrer Herstellung und ihre Verwendung als Zwischenprodukte zur Synthese von optisch aktiven Wirkstoffen mit fungiziden und pflanzenwuchsregulierenden Eigenschaften.

Es sind bereits Racemate von zahlreichen Oxiranen bekannt (vgl. EP-OS 0 040 345, EP-OS 0 052 424, EP-OS 0 061 835 und EP-OS 0 084 834). Die entsprechenden Oxirane sind jedoch wegen der Reaktivität des Oxiranringes nicht ohne Schwierigkeiten zugänglich.

Weiterhin ist bekannt, daß sich Oxirane durch Umsetzung der zugrunde liegenden Ketone mit Sulfonium- oder Sulfoxonium-yliden herstellen lassen (vgl. EP-OS 0 040 345, EP-OS 0 124 009, EP-OS 0 124 011, EP-OS 0 124 013 und EP-OS 0 124 014). Nachteilig an den bekannten Verfahren ist aber, daß danach keine optisch aktiven Oxirane stereospezifisch zugänglich sind.

Es wurden nun neue optisch aktive Oxirane der Formel

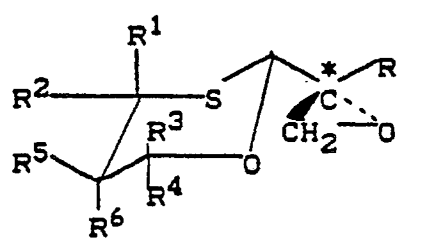

(I)

in welcher

R für gegebenenfalls substituiertes Alkyl, gegebenenfalls substituiertes Cycloalkyl oder für gegebenenfalls substituiertes Phenyl steht und

$R^1$, $R^2$, $R^3$, $R^4$, $R^5$ und $R^6$ für Wasserstoff oder Alkyl stehen, wobei jedoch mindestens einer der Reste für Alkyl steht, oder

$R^4$ und $R^5$ gemeinsam für gegebenenfalls durch Alkyl substituiertes Alkandiyl stehen oder

$R^4$ und $R^5$ gemeinsam mit den angrenzenden Kohlenstoffatomen für einen gegebenenfalls durch Alkyl substituierten, anellierten bicyclischen Kohlenwasserstoff-Rest stehen,

gefunden.

Weiterhin wurde gefunden, daß sich optisch aktive Oxirane der Formel (I) herstellen lassen, indem man enantiomeren-reine Oxathian-Ketone der Formel

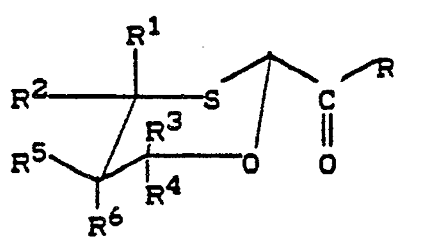

(II)

in welcher

R, $R^1$, $R^2$, $R^3$, $R^4$, $R^5$ und $R^6$ die oben angegebene Bedeutung haben,

entweder α) mit Sulfonium-Salzen der Formel

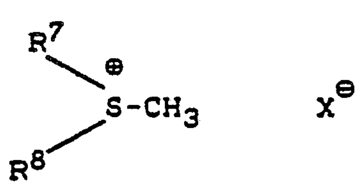

(III)

in welcher

$R^7$ für Methyl oder Phenyl steht,
$R^8$ für Methyl oder Phenyl steht und
$X^{\ominus}$ für Halogenid oder Methosulfat steht,
oder
ß) mit Sulfoxonium-Salzen der Formel

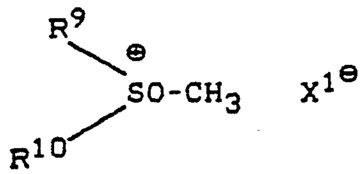
(IV)

in welcher
$R^9$ für Methyl oder Phenyl steht,
$R^{10}$ für Methyl oder Phenyl steht und
$X^{1\ominus}$ für Halogenid oder Methosulfat steht,
in Gegenwart einer starken Base und in Gegenwart eines Verdünnungsmittels bei Temperaturen zwischen -78°C und +100°C umsetzt.

Schließlich wurde gefunden, daß sich optisch aktive Oxirane der Formel (I) als Zwischenprodukte zur Herstellung von optisch aktiven Wirkstoffen mit fungiziden und pflanzenwuchsregulierenden Eigenschaften verwenden lassen. So erhält man optisch aktive 2-Hydroxyethylazol-Derivate der Formel

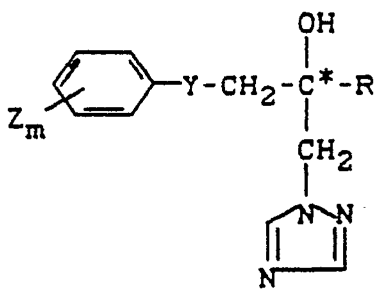
(V)

in welcher
R die oben angegebene Bedeutung hat,
Y für Sauerstoff, eine direkte Bindung oder für eine $CH_2$-Gruppe steht,
Z für Halogen, Alkyl, Cycloalkyl, Alkoxy, Alkylthio, Halogenalkyl, Halogenalkoxy, Halogenalkylthio, gegebenenfalls substituiertes Phenoxy, gegebenenfalls substituiertes Phenylalkyl oder gegebenenfalls substituiertes Phenoxyalkyl steht und
m für die Zahlen 0, 1, 2 oder 3 steht,
indem man optisch aktive Oxirane der Formel

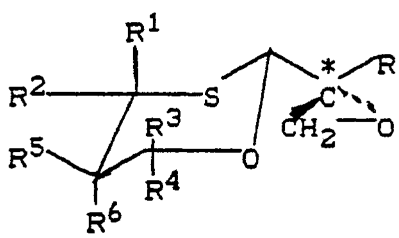
(I)

in welcher
R, $R^1$, $R^2$, $R^3$, $R^4$, $R^5$ und $R^6$ die oben angegebene Bedeutung haben,
in einer ersten Stufe entweder
γ) mit Phenol-Derivaten der Formel

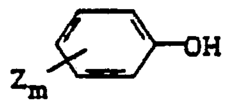

(VI)

in welcher
Z und m die oben angegebene Bedeutung haben,
in Gegenwart einer Base und in Gegenwart eines Verdünnungsmittel bei Temperaturen zwischen 0° C und 150° C umsetzt,
oder
δ) mit Grignard-Verbindungen der Formel

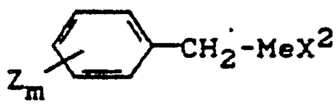

(VII)

in welcher
Z und m die oben angegebene Bedeutung haben,
Me für ein Erdalkalimetall oder für Zink steht und
$X^2$ für Chlor, Brom oder Iod steht,
in Gegenwart eines Verdünnungsmittels bei Temperaturen zwischen -78° C und +100° C umsetzt,
danach in einer zweiten Stufe die so erhaltenen optisch aktiven Verbindungen der Formel

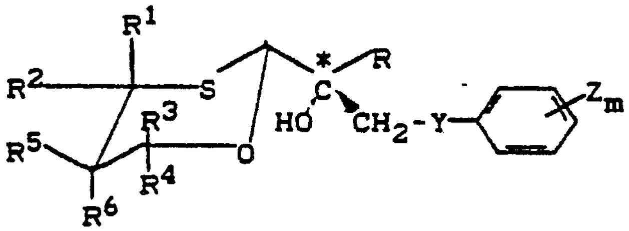

(VIII)

in welcher
R, $R^1$, $R^2$, $R^3$, $R^4$, $R^5$, $R^6$, Y, Z und m die oben angegebene Bedeutung haben,
mit einem zur Spaltung von Oxathian-Verbindungen geeigneten Reagenz in Gegenwart eines Verdünnungsmittels sowie gegebenenfalls in Gegenwart eines Säurebindemittels bei Temperaturen zwischen 0° C und 100° C umsetzt und die dabei entstehenden optisch aktiven α-Hydroxyaldehyde der Formel

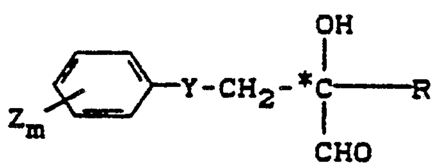

(IX)

in welcher
R, Y, Z und m die oben angegebene Bedeutung haben,
mit einem zur Reduktion von Aldehyden geeigneten Reagenz in Gegenwart eines Verdünnungsmittels bei Temperaturen zwischen -20° C und +100° C umsetzt,
weiterhin in einer dritten Stufe die so erhaltenen optisch aktiven Diole der Formel

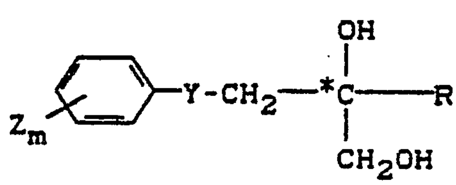

(X)

in welcher
R, Y, Z und m die oben angegebene Bedeutung haben,
mit Sulfonsäure-Derivaten der Formel
$R^{11}$-$SO_2$-Hal (XI)
in welcher
$R^{11}$ für Alkyl, Halogenalkyl oder gegebenenfalls substituiertes Phenyl steht und
Hal für Halogen steht,
in Gegenwart eines Verdünnungsmittels und in Gegenwart eines Säurebindemittels bei Temperaturen zwischen 0°C und 100°C umsetzt und schließlich in einer vierten Stufe die entstandenen optisch aktiven Verbindungen der Formel

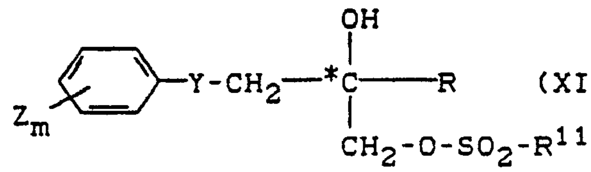 (XII)

in welcher
R, $R^{11}$, Y, Z und m die oben angegebene Bedeutung haben,
mit Triazol-Salzen der Formel

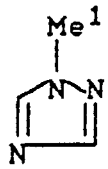 (XIII)

in welcher
$Me^1$ für ein Alkalimetall steht,
in Gegenwart eines Verdünnungsmittels und gegebenenfalls in Gegenwart eines Säurebindemittels bei Temperaturen zwischen 20°C und 150°C umsetzt.

Im vorliegenden Falle wird in den Formelzeichnungen die sterische Anordnung der einzelnen Gruppen nur zum Teil angedeutet. Ferner sind asymmetrisch substituierte Kohlenstoffatome dann durch ein (*) gekennzeichnet, wenn es sich um optisch aktive Verbindungen handelt. Außer den gekennzeichneten Kohlenstoffatomen können noch weitere asymmetrisch substituierte Kohlenstoffatome enthalten sein.

Der Verlauf des erfindungsgemäßen Verfahrens zur Herstellung von optisch aktiven Oxiranen der Formel (I) ist als äußerst überraschend zu bezeichnen. So konnte nicht erwartet werden, daß die sehr reaktiven Ylide, die sich bei der Behandlung von Sulfonium-Salzen der Formel (III) bzw. Sulfoxonium-Salzen der Formel (IV) mit starken Basen intermediär bilden, stereoselektiv mit Oxathian-Ketonen der Formel (II) reagieren. Vielmehr war davon auszugehen, daß die Schwefel-Ylide aufgrund ihrer hohen Reaktivität die Carbonyl-Gruppe so angreifen, daß Gemische entstehen, in denen die beiden Formen der optisch aktiven Oxirane der Formel (I) in nahezu gleichen Teilen enthalten sind.

Überraschend ist auch, daß sich die optisch aktiven Oxirane der Formel (I) mit hoher Selektivität und in sehr guter Ausbeute in optisch aktive 2-Hydroxyethylazol-Derivate der Formel (V) überführen lassen. Wegen der Mehrstufigkeit der Synthese war nämlich damit zu rechnen, daß Nebenreaktionen eintreten und in den Endprodukten jeweils neben dem gewünschten Enantiomeren auch das entsprechende unerwünschte Enantiomere in größerer Menge vorhanden ist.

Das erfindungsgemäße Verfahren zur Herstellung der optisch aktiven Oxirane der Formel (I) zeichnet sich durch eine Reihe von Vorteilen aus. So sind die als Ausgangsstoffe benötigten Oxathian-Ketone enantiomeren-rein zugänglich und ebenso wie die erforderlichen Reaktionskomponenten auch in größeren Mengen verfügbar. Weiterhin bereiten die Umsetzung und die Aufarbeitung keinerlei Schwierigkeiten. Der entscheidenste Vorteil besteht jedoch darin, daß die optisch aktiven Oxirane in direkter Synthese stereospezifisch herstellbar sind und keine Racemat-Trennungen vorgenommen werden müssen.

Das Verfahren zur Herstellung von optisch aktiven 2-Hydroxyethyl-azol-Derivaten der Formel (V) ist ebenfalls mit erheblichen Vorteilen verbunden. Auch hier werden als Reaktionskomponenten nur übliche Chemikalien eingesetzt, die auch in technischen Mengen verfügbar und leicht zu handhaben sind. Außerdem bereiten auch hier die einzelnen Umsetzungen und die Aufarbeitung der jeweils anfallenden Reaktionsprodukte keine Probleme. Besonders günstig ist schließlich, daß bei der asymmetrischen Totalsynthese der optisch aktiven 2-Hydroxyethyl-azol-Derivate der Formel (V) das gesamte Ausgangsmaterial in das jeweils gewünschte Enantiomere überführt wird.

Die erfindungsgemäßen optisch aktiven Oxirane sind durch die Formel (I) allgemein definiert. Bevorzugt sind diejenigen Stoffe der Formel (I), in denen

R für gegebenenfalls durch Halogen, Alkoxy mit 1 bis 4 Kohlenstoffatomen, Alkythio mit 1 bis 4 Kohlenstoffatomen, die CHO-Gruppe und deren Derivate, Phenoxy und/oder Phenyl substituiertes geradkettiges oder verzweigtes Alkyl mit 1 bis 6 Kohlenstoffatomen, für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen und/oder Halogen substituiertes Cycloalkyl mit 3 bis 8 Kohlenstoffatomen oder für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen und/oder Halogen substituiertes Phenyl steht und

$R^1$, $R^2$, $R^3$, $R^4$, $R^5$ und $R^6$ für Wasserstoff oder Alkyl mit 1 bis 4 Kohlenstoffatomen stehen, wobei jedoch mindestens einer dieser Reste für Alkyl mit 1 bis 4 Kohlenstoffatomen steht, und außerdem

$R^4$ und $R^5$ auch gemeinsam für gegebenenfalls ein- bis dreifach, gleichartig oder verschieden durch Alkyl mit 1 bis 4 Kohlenstoffatomen substituiertes Alkandiyl mit 3 bis 6 Kohlenstoffatomen stehen oder auch

$R^4$ und $R^5$ gemeinsam mit den angrenzenden Kohlenstoffatomen, für einen gegebenenfalls ein- bis dreifach, gleichartig oder verschieden durch Alkyl mit 1 bis 4 Kohlenstoffatomen substituierten, anellierten bicyclischen Kohlenwasserstoff mit 7 oder 8 Kohlenstoffatomen stehen.

Besonders bevorzugt sind diejenigen optisch aktiven Verbindungen der Formel (I), in denen

R für gegebenenfalls durch Fluor, Chlor, Brom, Alkoxy mit 1 oder 2 Kohlenstoffatomen, Dioxolanyl, Formyl, Methoximinomethyl, Alkylthio mit 1 oder 2 Kohlenstoffatomen, Phenoxy und/oder Phenyl substituiertes Alkyl mit 1 bis 4 Kohlenstoffatomen steht, für gegebenenfalls durch Alkyl mit 1 oder 2 Kohlenstoffatomen, Alkoxy mit 1 oder 2 Kohlenstoffatomen, Alkylthio mit 1 oder 2 Kohlenstoffatomen, Fluor, Chlor und/oder Brom substituiertes Cycloalkyl mit 3 bis 7 Kohlenstoffatomen steht oder für gegebenenfalls durch Alkyl mit 1 oder 2 Kohlenstoffatomen, Alkoxy mit 1 oder 2 Kohlenstoffatomen, Alkylthio mit 1 oder 2 Kohlenstoffatomen, Fluor, Chlor und/oder Brom substituiertes Phenyl steht und

$R^1$, $R^2$, $R^3$, $R^4$, $R^5$ und $R^6$ für Wasserstoff, Methyl oder Ethyl stehen, wobei jedoch mindestens einer dieser Reste für Methyl oder Ethyl steht, und außerdem $R^4$ und $R^5$ auch gemeinsam für gegebenenfalls ein- bis dreifach, gleichartig oder verschieden durch Methyl und/oder Ethyl substituiertes Alkandiyl mit 3 bis 5 Kohlenstoffatomen stehen oder auch

$R^4$ und $R^5$ gemeinsam mit den angrenzenden Kohlenstoffatomen für einen gegebenenfalls ein- bis dreifach, gleichartig oder verschieden durch Methyl und/oder Ethyl substituierten, anellierten bicyclischen Kohlenwasserstoff mit 7 oder 8 Kohlenstoffatomen stehen.

Ganz besonders bevorzugt sind diejenigen optisch aktiven Oxirane der Formel (I), in denen

R für gegebenenfalls ein- oder zweifach, gleichartig oder verschieden durch Fluor, Chlor, Brom, Methoxy, Methylthio, Methoximinomethyl, Phenoxy und/oder Phenyl substituiertes Methyl, Ethyl, Isopropyl oder tert.-Butyl steht, weiterhin für die Gruppierung

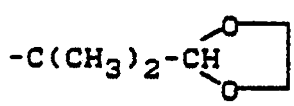 steht,

ferner für jeweils gegebenenfalls ein- oder zweifach, gleichartig oder verschieden durch Methyl, Methylthio, Fluor und/oder Chlor substituiertes Cyclopropyl, Cyclopentyl oder Cyclohexyl steht oder für gegebenenfalls ein- oder zweifach, gleichartig oder verschieden durch Methyl, Methoxy, Methylthio, Fluor und/oder Chlor substituiertes Phenyl steht und

der Oxathian-Teil für die Gruppierungen der Formeln

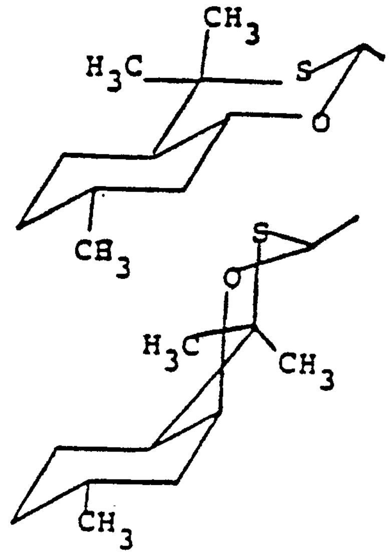

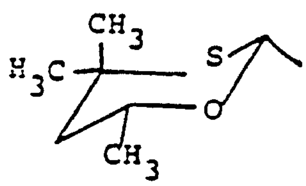

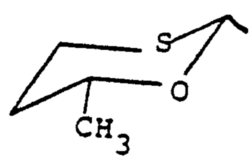

oder

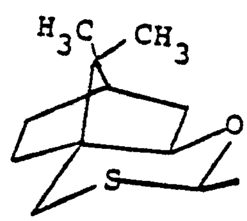

steht.

Verwendet man bei der Durchführung des erfindungsgemäßen Verfahrens enantiomeren-reines Oxathian-Keton der Formel (II-1) als Ausgangsstoff, Trimethyl-sulfoxonium-iodid als Reaktionskomponente und Kalium-tert.-butylat als Base, so kann der Verlauf der Umsetzung durch das folgende Formelschema veranschaulicht werden:

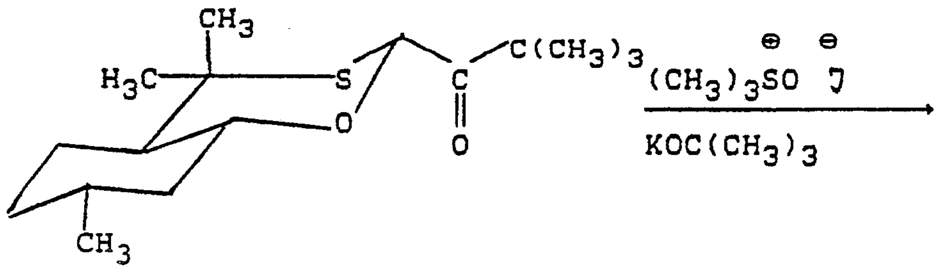


(II-1)

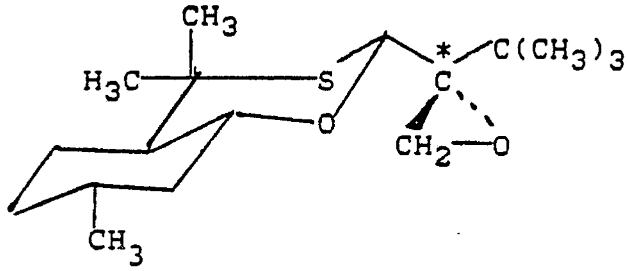

Diastereomer (A) (I-1)

Welches der beiden möglichen Diastereomeren (A) und (B) bei der erfindungsgemäßen Umsetzung entsteht, hängt von der sterischen Struktur des eingesetzten Oxathian-Ketons ab.

Die bei der Durchführung des erfindungsgemäßen Verfahrens als Ausgangsstoffe benötigten enantiomeren-reinen Oxathian-Ketone sind durch die Formel (II) allgemein definiert. In dieser Formel haben R, $R^1$, $R^2$, $R^3$, $R^4$, $R^5$ und $R^6$ vorzugsweise diejenigen Bedeutungen, die bereits im Zusammenhang mit der Beschreibung der erfindungsgemäßen Stoffe der Formel (I) vorzugsweise für diese Reste genannt wurden. Die enantiomeren-reinen Oxathian-Ketone der Formel (II) sind bekannt oder lassen sich nach prinzipiell bekannten Methoden herstellen (vgl. DE-OS 36 27 673).

Die bei der Durchführung des erfindungsgemäßen Verfahrens (Variante α) als Reaktionskomponenten benötigten Sulfonium-Salze sind durch die Formel (III) allgemein definiert. Bevorzugt sind diejenigen Verbindungen der Formel (III), in denen

$R^7$ für Methyl oder Phenyl steht,

$R^8$ für Methyl oder Phenyl steht und

$X^\ominus$ für Chlorid, Bromid, Iodid oder Methosulfat steht,

Die bei der Durchführung des erfindungsgemäßen Verfahrens (Variante ß) als Reaktionskomponenten benötigten Sulfoxonium-Salze sind durch die Formel (IV) allgemein definiert. Bevorzugt sind diejenigen Verbindungen der Formel (IV), in denen

$R^9$ für Methyl oder Phenyl steht,

$R^{10}$ für Methyl oder Phenyl steht und

$X'^\ominus$ für Chlorid, Bromid, Iodid oder Methosulfat steht.

Sowohl die Sulfonium-Salze der Formel (III) als auch die Sulfoxonium-Salze der Formel (IV) sind bekannt oder lassen sich nach bekannten Methoden herstellen (vgl. J. Amer. Chem. Soc. 87, 1353-1364 (1965) und EP-OS 0 040 345).

Als Basen können bei der Durchführung des erfindungsgemäßen Verfahrens sowohl bei der Variante α als auch bei der Variante ß alle üblichen starken anorganischen und organischen Basen verwendet werden. Vorzugsweise in Betracht kommen Natriumhydrid, Natriumamid und Alkalihydroxide, wie zum Beispiel Natriumhydroxid oder Kaliumhydroxid, und außerdem Alkalimetallalkoholate, wie Natriummethylat, Natriumethylat und Kalium-tert.-butylat.

Als Verdünnungsmittel können bei der Durchführung des erfindungsgemäßen Verfahrens sowohl beim Arbeiten nach der Variante α als auch nach der Variante ß alle für derartige Umsetzungen üblichen inerten organischen Lösungsmittel verwendet werden. Vorzugsweise in Frage kommen Nitrile, wie Acetonitril, ferner Alkohole, wie Methanol, Ethanol, Propanol, n-Butanol und tert.-Butanol, außerdem polare Solventien, wie Dimethylsulfoxid, weiterhin aromatische oder aliphatische Kohlenwasserstoffe, wie Hexan, Benzol, Toluol und Xylol, und schließlich auch Dimethylsulfid oder Dimethylsulfat.

Die Reaktionstemperaturen können bei der Durchführung des erfindungsgemäßen Verfahrens sowohl

beim Arbeiten nach der Variante α als auch nach der Variante ß innerhalb eines bestimmten Bereiches variiert werden. Im allgemeinen arbeitet man bei Temperaturen zwischen -78° C und +100° C, vorzugsweise zwischen 0° C und 60° C.

Das erfindungsgemäße Verfahren wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens werden die Mengen an Reaktionskomponenten so gewählt, daß auf 1 Mol an enantiomeren-reinem Oxathian-Keton der Formel (II) im allgemeinen 1,0 bis 2,0 Mol, vorzugsweise 1,1 bis 1,8 Mol an Sulfonium-Salz der Formel (III) bzw. an Sulfoxonium-Salz der Formel (IV) und 1,0 bis 2,5 Mol, vorzugsweise 1,3 bis 2,0 Mol an Base vorhanden sind.

Im einzelnen verfährt man bei der Durchführung des erfindungsgemäßen Verfahrens in der Weise, daß man ein Gemisch aus enantiomeren-reinem Oxathian-Keton der Formel (II) und frisch hergestelltem Sulfonium-Salz der Formel (III) oder Sulfoxonium-Salz der Formel (IV) in einem Verdünnungsmittels gegebenenfalls unter Schutzgasatmosphäre mit starker Base versetzt und rührt. Die anschließende Aufarbeitung erfolgt nach üblichen Methoden. Im allgemeinen geht man so vor, daß man das Reaktionsgemisch zunächst durch Abziehen des Verdünnungsmittels einengt, den Rückstand mit Wasser behandelt, das entstehende Gemisch mit einem mit Wasser wenig mischbaren organischen Solvens extrahiert und die vereinigten organischen Phasen trocknet und einengt. Das anfallende Produkt kann im Bedarfsfall nach üblichen Methoden, zum Beispiel durch säulenchromatographische Reinigung, von eventuell noch enthaltenen unerwünschten Substanzen befreit werden.

Die erfindungsgemäßen optisch aktiven Oxirane der Formel (I) sind, wie schon erwähnt, wertvolle Zwischenprodukte zur Synthese von optisch aktiven 2-Hydroxyethyl-azol-Derivaten mit fungiziden und pflanzenwuchsregulierenden Eigenschaften.

Verwendet man bei der Durchführung der Variante γ des Verfahrens zur Herstellung von Verbindungen der Formel (V) optisch aktives Oxiran der Formel (I-1) und 4-Chlorphenol als Ausgangsstoffe, N-Chlorsuccinimid und Silbernitrat als Reagenz zur Spaltung der Oxathian-Verbindung in der zweiten Stufe und Lithiumaluminiumhydrid zur Reduktion des Aldehyds in der zweiten Stufe, Chlormethansulfonsäure als Reaktionskomponente in der dritten Stufe und 1,2,4-Triazol-Natriumsalz als Reaktionspartner in der vierten Stufe, so kann der Reaktionsablauf durch das folgende Formelschema veranschaulicht werden:

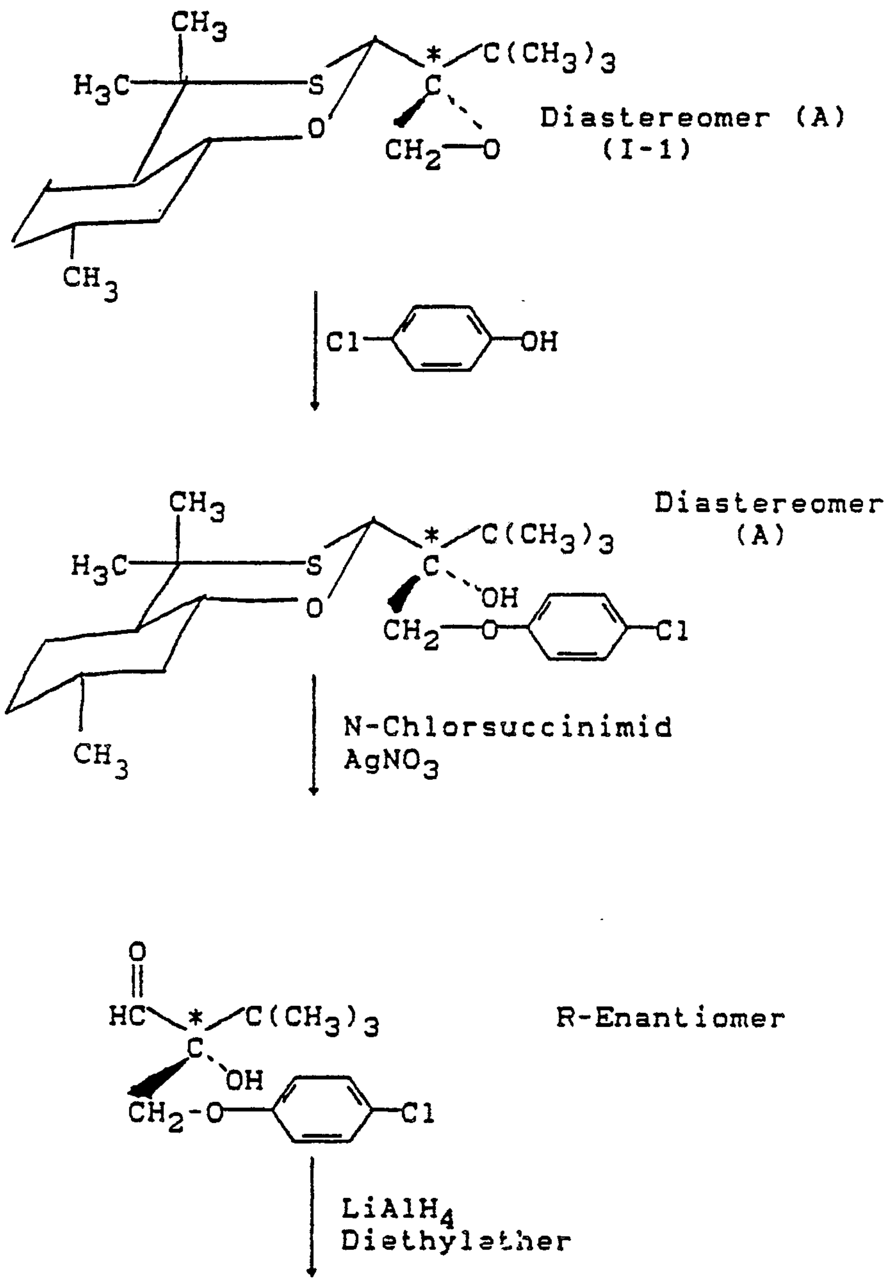


Diastereomer (A)
(I-1)

Diastereomer
(A)

R-Enantiomer

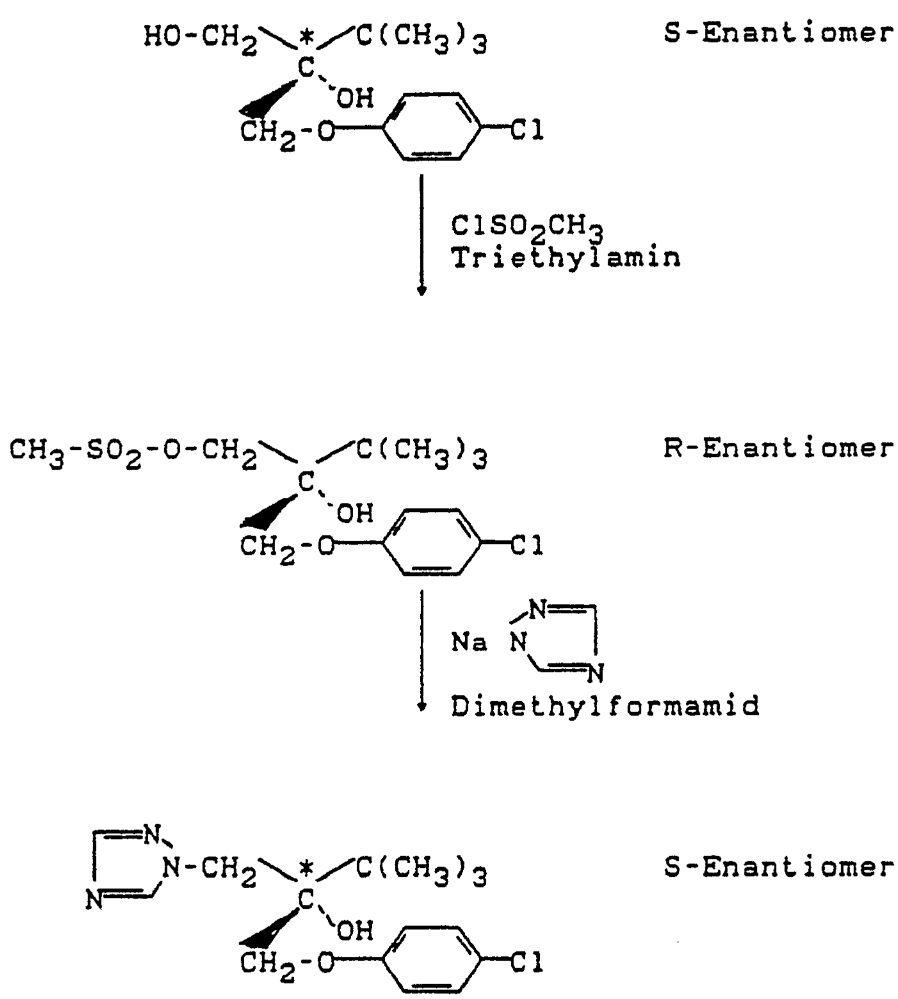


Verwendet man bei der Durchführung der Variante δ des Verfahrens zur Herstellung von Verbindungen der Formel (V) optisch aktives Oxiran der Formel (I-1) und 4-Chlorbenzyl-Magnesiumbromid als Ausgangsstoffe, N-Chlorsuccinimid und Silbernitrat als Reagenz zur Spaltung der Oxathian-Verbindung in der zweiten Stufe und Lithiumaluminiumhydrid zur Reduktion des Aldehyds in der zweiten Stufe, Chlormethansulfonsäure als Reaktionskomponente in der dritten Stufe und 1,2,4-Triazol-Natriumsalz als Reaktionspartner in der vierten Stufe, so kann der Reaktionsablauf durch das folgende Formelschema veranschaulicht werden:

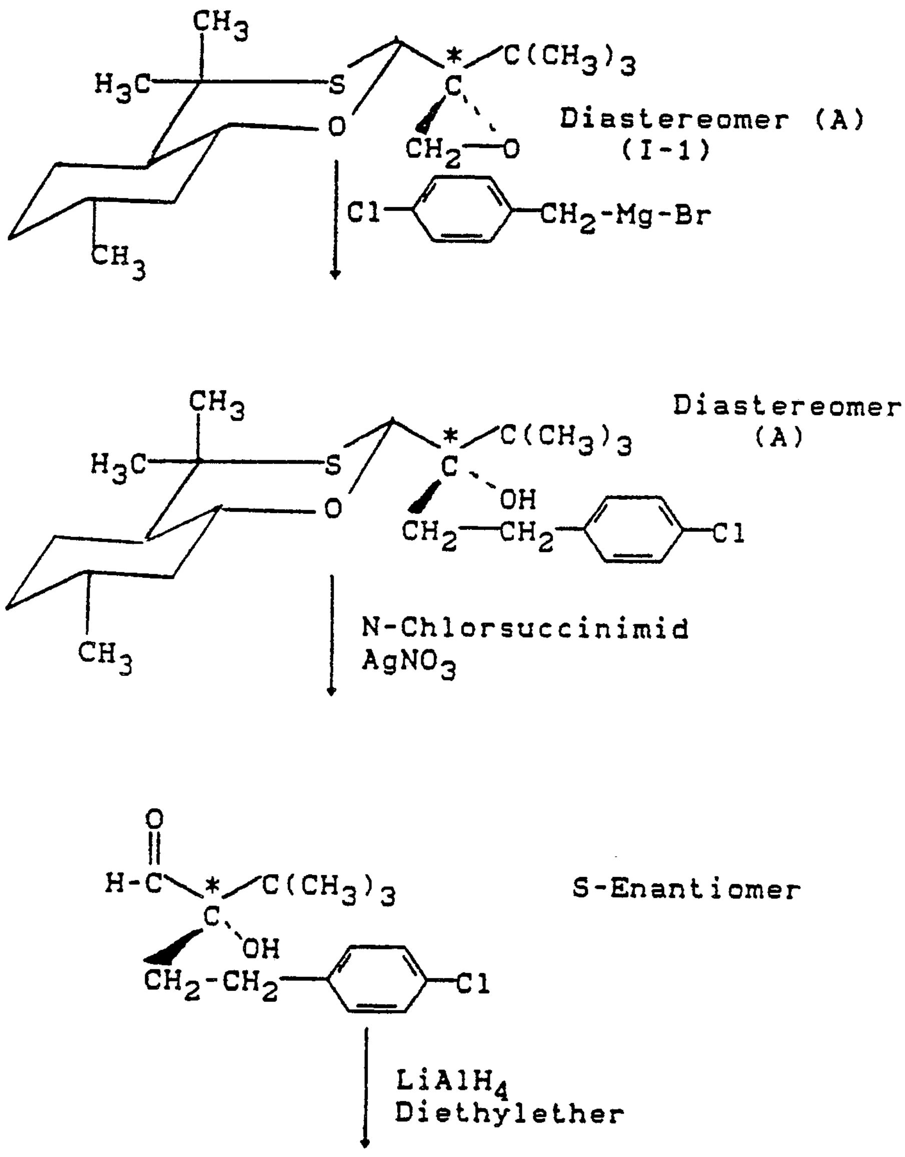


Diastereomer (A) (I-1)

Diastereomer (A)

S-Enantiomer

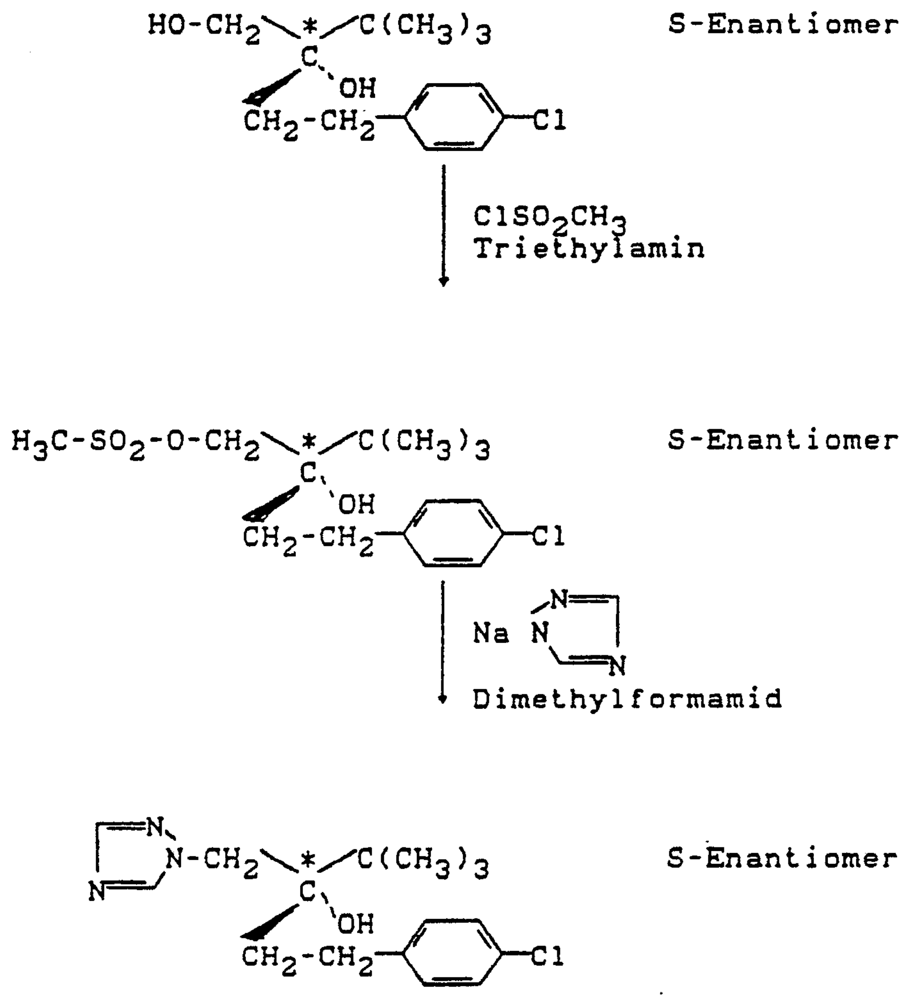


In den obigen Formelzeichnungen und auch im folgenden Teil der Beschreibung bezieht sich die Konfigurationszuordnung "R" bzw. "S" jeweils auf das Carbinol-Kohlenstoffatom.

Die bei der Durchführung der Variante $\gamma$ des Verfahrens zur Herstellung von optisch aktiven 2-Hydroxyethyl-azol-Derivaten der Formel (V) als Reaktionskomponenten benötigten Phenol-Derivate sind durch die Formel (VI) allgemein definiert. Bevorzugt sind Verbindungen der Formel (VI), in denen

Z für Fluor, Chlor, Brom, Alkyl mit 1 bis 4 Kohlenstoffatomen, Cycloalkyl mit 3 bis 8 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht.

Besonders bevorzugt sind diejenigen Phenol-Derivate der Formel (VI), in denen

Z für Fluor, Chlor, Brom, Alkyl mit 1 bis 4 Kohlenstoffatomen, Cycloalkyl mit 3 bis 7 Kohlenstoffatomen, Alkoxy mit 1 oder 2 Kohlenstoffatomen, Alkylthio mit 1 oder 2 Kohlenstoffatomen, Halogenmethyl mit 1 bis 3 gleichen oder verschiedenen Halogenatomen, Halogenmethoxy mit 1 bis 3 gleichen oder verschiedenen Halogenatomen, Halogenmethylthio mit 1 bis 3 gleichen oder verschiedenen Halogenatomen, gegebenenfalls durch Fluor, Chlor und/oder Methyl substituiertes Phenyl, gegebenenfalls durch Fluor, Chlor und/oder Methyl substituiertes Phenoxy, gegebenenfalls durch Fluor, Chlor und/oder Methyl substituiertes Benzyl oder für gegebenenfalls durch Fluor, Chlor und/oder Methyl substituiertes Benzyloxy steht und

m für die Zahlen 0, 1, 2 oder 3 steht.

Ganz besonders bevorzugt sind diejenigen Stoffe der Formel (VI), in denen

Z für Fluor, Chlor, Brom, Methyl, Ethyl, Isopropyl, tert.-Butyl, Cyclopropyl, Cyclopentyl, Cyclohexyl, Methoxy, Methylthio, Trifluormethyl, Trifluormethoxy, Trifluormethylthio, gegebenenfalls ein- oder zweifach, gleichartig oder verschieden durch Fluor, Chlor und/oder Methyl substituiertes Phenyl, gegebenenfalls ein- oder zweifach, gleichartig oder verschieden durch Fluor, Chlor und/oder Methyl substituiertes Phenoxy, gegebenenfalls ein- oder zweifach, gleichartig oder verschieden durch Fluor, Chlor und/oder Methyl substituiertes Benzyl oder für gegebenenfalls ein- oder zweifach, gleichartig oder verschieden durch Fluor, Chlor und/oder Methyl substituiertes Benzyloxy steht und

m für die Zahlen 0, 1, 2 oder 3 steht.

Die Phenol-Derivate der Formel (VI) sind allgemein bekannte Verbindungen der organischen Chemie.

Als Basen können bei der Durchführung der Variante $\gamma$ des Verfahrens zur Herstellung der optisch aktiven Verbindungen der Formel (V) alle für derartige Umsetzungen üblichen Säurebinder verwendet werden. Vorzugsweise in Betracht kommen Hydride, wie Natriumhydrid, ferner Alkalimetallhydroxide und Alkalimetallcarbonate, wie Natriumhydroxid, Kaliumhydroxid, Natriumcarbonat und Kaliumcarbonat, und außerdem auch Alkalimetallalkoholate, wie Natriummethylat, Natriumethylat und Kaliumtert.-butylat.

Als Verdünnungsmittel können bei der Durchführung der Variante $\gamma$ des Verfahrens zur Herstellung der optisch aktiven Verbindungen der Formel (V) alle für derartige Umsetzungen üblichen inerten organischen Solventien verwendet werden. Vorzugsweise in Frage kommen Alkohole, wie Methanol, Ethanol, n-Propanol, Isopropanol, n-Butanol und tert.-Butanol, und außerdem stark polare Lösungsmittel, wie Dimethylformamid, Dimethylsulfoxid und Acetonitril.

Die Reaktionstemperaturen können bei der Durchführung der Variante $\gamma$ des Verfahrens zur Herstellung der optisch aktiven Verbindungen der Formel (V) innerhalb eines größeren Bereiches variiert werden. Im allgemeinen arbeitet man bei Temperaturen zwischen 0°C und 150°C, vorzugsweise zwischen 10°C und 130°C.

Die Umsetzung nach der Variante $\gamma$ des Verfahrens zur Herstellung der optisch aktiven Verbindungen der Formel (V) wird im allgemeinen unter Normaldruck durchgeführt. Es ist jedoch auch möglich, unter erhöhtem oder vermindertem Druck zu arbeiten.

Bei der Durchführung der Variante $\gamma$ des Verfahrens zur Herstellung der optisch aktiven Verbindungen der Formel (V) setzt man auf 1 Mol an optisch aktivem Oxiran der Formel (I) im allgemeinen 1 bis 5 Mol, vorzugsweise 2 bis 4 Mol an Phenol-Derivat der Formel (VI) sowie 1 bis 5 Mol, vorzugsweise 2,5 bis 4 Mol an Base ein. Die Aufarbeitung erfolgt nach üblichen Methoden. Im allgemeinen geht man so vor, daß man das Reaktionsgemisch nach vorherigem Einengen mit Wasser behandelt, dann mit einem mit Wasser wenig mischbaren organischen Lösungsmittel extrahiert, die vereinigten organischen Phasen wäscht, danach trocknet und durch Abziehen des Verdünnungsmittels unter vermindertem Druck einengt und gegebenenfalls den verbleibenden Rückstand nach üblichen Methoden reinigt.

Die bei der Durchführung der Variante $\delta$ des Verfahrens zur Herstellung von optisch aktiven Verbindungen der Formel (V) als Reaktionskomponenten benötigten Grignard-Verbindungen sind durch die Formel (VII) allgemein definiert. In dieser Formel haben Z und m vorzugsweise diejenigen Bedeutungen, die bereits im Zusammenhang mit der Beschreibung der Phenol-Derivate der Formel (VI) für diesen Rest und diesen Index als bevorzugt genannt wurden. Me Steht vorzugsweise für Magnesium oder Zink, und $X^2$ steht für Chlor, Brom oder Iod.

Die Grignard-Verbindungen der Formel (VII) sind bekannt oder lassen sich nach prinzipiell bekannten Verfahren herstellen.

Als Verdünnungsmittel kommen bei der Durchführung der Variante $\delta$ zur Herstellung von optisch aktiven Verbindungen der Formel (V) alle für derartige Umsetzungen üblichen organischen Solventien in Frage. Vorzugsweise verwendbar sind Ether, wie Diethylether, Tetrahydrofuran und Dioxan.

Die Reaktionstemperaturen können bei der Durchführung der Variante $\delta$ des Verfahrens zur Herstellung von optisch aktiven Verbindungen der Formel (V) innerhalb eines bestimmten Bereiches variiert werden. Im allgemeinen arbeitet man bei Temperaturen zwischen -78°C und +100°C, vorzugsweise zwischen -78°C und 90°C.

Die Umsetzung nach der Variante $\delta$ des Verfahrens zur Herstellung der optisch aktiven Verbindungen der Formel (V) wird im allgemeinen unter Normaldruck durchgeführt.

Bei der Durchführung der Variante $\delta$ des Verfahrens zur Herstellung der optisch aktiven Verbindungen der Formel (V) setzt man auf 1 Mol an optisch aktivem Oxiran der Formel (I) im allgemeinen 1 bis 3 Mol, vorzugsweise 1 bis 1,5 Mol an Grignard-Verbindung der Formel (VII) ein. Die Aufarbeitung erfolgt nach üblichen Methoden. Im allgemeinen geht man in der Weise vor, daß man das Reaktionsgemisch mit wäßriger Ammoniumchlorid-Lösung versetzt, die organische Phase abtrennt, die wäßrige Phase mehrfach mit einem mit Wasser wenig mischbaren organischen Solvens extrahiert, die vereinigten organischen

Phasen, trocknet, einengt und unter vermindertem Druck andestilliert. Eine eventuell erforderliche weitere Reinigung kann nach üblichen Methoden durchgeführt werden.

Bei der Durchführung der zweiten Stufe des Verfahrens zur Herstellung von optisch aktiven Verbindungen der Formel (V) kommen als Reagenzien zur Spaltung der Verbindungen der Formel (VIII) alle für derartige Zwecke üblichen Substanzen in Frage. Vorzugsweise verwendbar sind Gemische aus N-Chlor-succinimid und Silbernitrat.

Als Verdünnungsmittel kommen bei der Durchführung der Spaltung der Oxathian-Verbindungen der Formel (VIII) alle für derartige Reaktionen üblichen Solventien in Betracht. Vorzugsweise verwendbar sind Gemische aus Wasser und polaren organischen Verdünnungsmitteln, die mit Wasser mischbar sind. Beispielhaft genannte seien Gemische aus Acetonitril und Wasser.

Als Säurebindemittel können bei der Durchführung der Spaltung der Oxathian-Verbindungen der Formel (VIII) alle für derartige Umsetzungen üblichen Säureakzeptoren eingesetzt werden. Vorzugsweise verwendbar sind Alkalimetallcarbonate und Alkalimetallhydrogencarbonate, wie zum Beispiel Natriumhydrogencarbonat.

Die Reaktionstemperaturen können bei der Durchführung der Spaltung der Oxathian-Verbindungen der Formel (VIII) in der zweiten Stufe des Verfahrens zur Herstellung der optisch aktiven Verbindungen der Formel (V) in einem größeren Bereich variiert werden. Im allgemeinen arbeitet man bei Temperaturen zwischen 0° C und 100° C, vorzugsweise zwischen 20° C und 60° C.

Bei der Durchführung der Spaltung der Oxathian-Verbindungen der Formel (VIII) setzt man auf 1 Mol an einer Verbindung der Formel (VIII) im allgemeinen 1 bis 3 Mol an Spaltungsreagenz sowie einen Überschuß an Säurebindemittel ein. Die Aufarbeitung erfolgt nach üblichen Methoden. Im allgemeinen geht man so vor, daß man das Reaktionsgemisch mit wäßrigen Salzlösungen versetzt, die festen Bestandteile abfiltriert, das Filtrat mehrfach mit in Wasser wenig löslichen organischen Solventien extrahiert, die vereinigten organischen Phasen wäscht, trocknet und einengt. Das anfallende Produkt kann ohne zusätzliche Reinigung für die weiteren Umsetzungen verwendet werden.

Bei der Durchführung der zweiten Stufe des Verfahrens zur Herstellung von optisch aktiven Verbindungen der Formel (V) kommen als Reagenzien zur Reduktion von optisch aktiven α-Hydroxyaldehyden der Formel (IX) vorzugsweise komplexe Hybride, wie Lithiumaluminiumhydrid und Natriumborhydrid, in Betracht.

Als Verdünnungsmittel kommen bei der Reduktion von optisch aktiven α-Hydroxyaldehyden der Formel (IX) alle inerten organischen Solventien in Frage. Vorzugsweise verwendbar sind Ether, wie Diethylether oder Tetrahydrofuran. Arbeitet man mit Natriumborhydrid als Reduktionsmittel, so ist es auch möglich, Alkohole, wie Methanol oder Ethanol, oder Nitrile, wie Acetonitril, gegebenenfalls im Gemisch mit Wasser, als Verdünnungsmittel einzusetzen.

Die Reaktionstemperaturen können bei der Durchführung der Reduktion in der zweiten Stufe des Verfahrens zur Herstellung von optisch aktiven Verbindungen der Formel (V) innerhalb eines bestimmten Bereiches variiert werden. Im allgemeinen arbeitet man bei Temperaturen zwischen -20° C und +100° C, vorzugsweise zwischen 20° C und 60° C.

Bei der Durchführung der Reduktion in der zweiten Stufe des Verfahrens zur Herstellung von optisch aktiven Verbindungen der Formel (V) setzt man auf 1 Mol an optisch aktivem α-Hydroxyaldehyd der Formel (IX) im allgemeinen 1 bis 5 Mol an Reduktionsmittel ein. Die Aufarbeitung erfolgt nach üblichen Methoden. Im allgemeinen geht man in der Weise vor, daß man das Reaktionsgemisch mit einem in Wasser wenig löslichen organischen Solvens sowie mit wäßriger Alkalilauge versetzt, die vorhandenen festen Bestandteile absaugt und die organische Phase nach dem Trocknen einengt. Es ist jedoch auch möglich, das Reaktionsgemisch nach beendeter Umsetzung zunächst einzuengen, das verbleibende Produkt mit einem in Wasser wenig löslichen organischen Solvens zu extrahieren, die vereinigten organischen Phasen zu trocknen und anschließend einzuengen. Das anfallende Produkt kann, gegebenenfalls nach vorheriger Reinigung, für die weiteren Umsetzungen verwendet werden.

Die bei der Durchführung der dritten Stufe des Verfahrens zur Herstellung von optisch aktiven Verbindungen der Formel (V) als Reaktionskomponenten benötigten Sulfonsäure-Derivate sind durch die Formel (XI) allgemein definiert. Vorzugsweise verwendbar sind Sulfonsäure-Derivate der Formel (XI), in denen

$R^{11}$ für Alkyl mit 1 bis 4 Kohlenstoffatomen, Halogenalkyl mit 1 oder 2 Kohlenstoffatomen und 1 bis 5 Halogenatomen oder für gegebenenfalls durch Methyl substituiertes Phenyl steht und

Hal für Chlor oder Brom steht.

Als Beispiele für Sulfonsäure-Derivate der Formel (XI) seien genannt: Methansulfonsäurechlorid, Ethansulfonsäurechlorid, Trifluormethan-sulfonsäurechlorid und p-Tolylsulfonsäurechlorid.

Die Sulfonsäure-Derivate der Formel (XI) sind allgemein bekannte Verbindungen der organischen

Chemie.

Als Säurebindemittel kommen bei der Durchführung der dritten Stufe des Verfahrens zur Herstellung von optisch aktiven Verbindungen der Formel (V) vorzugsweise niedere tertiäre Alkylamine, Cycloalkylamine, Aralkylamine oder Arylamine in Betracht. Als Beispiele genannt seien Triethylamin, N,N-Dimethylbenzylamin, Pyridin, 1,4-Diazabicyclo[2,2,2]-octan und 1,5-Diazabicyclo[4,3,0]non-5-en.

Als Verdünnungsmittel kommen bei der Durchführung der dritten Stufe des Verfahrens zur Herstellung von optisch aktiven Verbindungen der Formel (V) alle für derartige Umsetzungen üblichen organischen Solventien in Frage. Vorzugsweise verwendbar sind halogenierte Kohlenwasserstoffe, wie Methylenchlorid, Chloroform und Tetrachlorkohlenstoff.

Die Reaktionstemperaturen können bei der Durchführung der dritten Stufe des Verfahrens zur Herstellung von optisch aktiven Verbindungen der Formel (V) innerhalb eines bestimmten Bereiches variiert werden. Im allgemeinen arbeitet man bei Temperaturen zwischen 0 °C und 100 °C, vorzugsweise zwischen 0 °C und 50 °C.

Bei der Durchführung der dritten Stufe des Verfahrens zur Herstellung von optisch aktiven Verbindungen der Formel (V) setzt man auf 1 Mol an optisch aktivem Diol der Formel (X) im allgemeinen 1 bis 1,3 Mol an Sulfonsäure-Derivat der Formel (XI) sowie 1 bis 1,3 Mol an Säurebindemittel ein. Die Aufarbeitung erfolgt nach üblichen Methoden. Im allgemeinen geht man so vor, daß man das Reaktionsgemisch zunächst mit schwach saurer und danach mit schwach basischer wäßriger Lösung wäscht, dann trocknet und einengt. Das anfallende Produkt kann ohne zusätzliche Reinigung für die weitere Umsetzung verwendet werden.

Die bei der Durchführung der vierten Stufe des Verfahrens zur Herstellung von optisch aktiven Verbindungen der Formel (V) als Reaktionskomponenten benötigten Triazol-Salze sind durch die Formel (XIII) definiert. Bevorzugt sind diejenigen Stoffe, in denen $Me^1$ für Natrium oder Kalium steht.

Die Triazol-Salze der Formel (XIII) sind bekannt.

Als Säurebindemittel können bei der Durchführung der vierten Stufe des Verfahrens zur Herstellung von optisch aktiven Verbindungen der Formel (V) alle üblichen organischen und anorganischen Basen eingesetzt werden.

Vorzugsweise verwendbar sind Alkalicarbonate, wie beispielsweise Natriumcarbonat oder Natriumhydrogencarbonat, ferner niedere tertiäre Alkylamine, Cycloalkylamine, Arylalkylamine oder Arylamine, wie beispielsweise Triethylamin, N,N-Dimethylbenzylamin, Pyridin, 1,4-Diazabicyclo[2,2,2]-octan oder 1,5-Diazabicyclo-[4.3.0]-non-5-en.

Als Verdünnungsmittel kommen bei der Durchführung der vierten Stufe des Verfahrens zur Herstellung von optisch aktiven Verbindungen der Formel (V) alle für derartige Umsetzungen üblichen inerten organischen Solventien in Frage. Vorzugsweise verwendbar sind polare, aprotische Verdünnungsmittel, wie Dimethylformamid und Dimethylsulfoxid.

Die Reaktionstemperaturen können bei der Durchführung der vierten Stufe des Verfahrens zur Herstellung von optisch aktiven Verbindungen der Formel (V) in einem größeren Bereich variiert werden. Im allgemeinen arbeitet man bei Temperaturen zwischen 20 °C und 150 °C, vorzugsweise zwischen 50 °C und 150 °C.

Die Umsetzungen in der vierten Stufe des Verfahrens zur Herstellung von optisch aktiven Verbindungen der Formel (V) werden gegebenenfalls unter Schutzgasatmosphäre, wie zum Beispiel unter Stickstoff oder Argon, durchgeführt.

Bei der Durchführung der vierten Stufe des Verfahrens zur Herstellung von optisch aktiven Verbindungen der Formel (V) setzt man auf 1 Mol an optisch aktiver Verbindung der Formel (XII) im allgemeinen 2 bis 4 Mol an Triazol-Salz der Formel (XIII) ein. Die Aufarbeitung erfolgt nach üblichen Methoden. Im allgemeinen geht man so vor, daß man das Reaktionsgemisch, gegebenenfalls nach vorherigem Abziehen des Verdünnungsmittels, mit Wasser sowie mit einem in Wasser wenig löslischen organischen Solvens versetzt, die organische Phase abtrennt, trocknet und einengt und den verbleibenden Rückstand auf chromatographischem Weg reinigt.

Die Durchführung der erfindungsgemäßen Verfahren wird durch die folgenden Beispiele veranschaulicht.

Beispiel 1

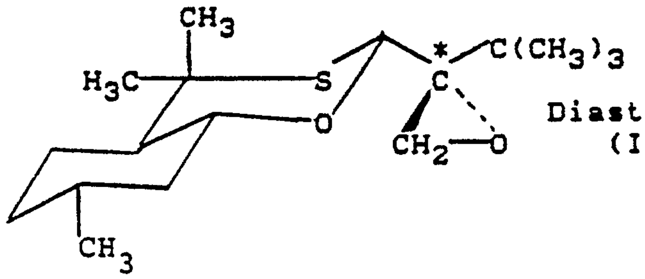

Diastereomer (A)
(I-1)

Zu einer Lösung von 25,0 g (88 mmol) Oxathian-Keton der Formel

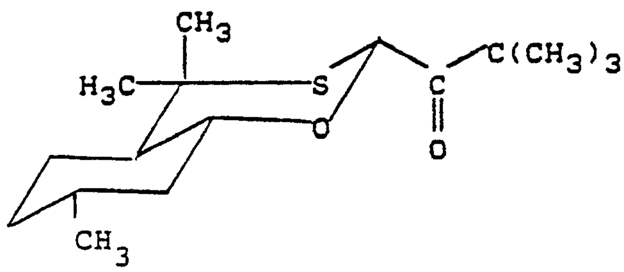

(II-1)

und 28,6 g (130 mmol) Trimethylsulfoxonium-iodid in 150 ml absolutem tert.-Butanol werden bei einer Temperatur von 10 °C unter Stickstoffatmosphäre und unter Rühren 14,56 g (130 mmol) Kalium-tert.-butylat portionsweise zugegeben. Danach wird noch 1 Stunde bei 10 °C und dann 16 Stunden bei Raumtemperatur gerührt. Zur Aufarbeitung wird das Reaktionsgemisch unter vermindertem Druck eingeengt, der verbleibende Rückstand in Wasser gegossen und das entstehende Gemisch mit Dichlormethan extrahiert. Die vereinigten organischen Phasen werden über Natriumsulfat getrocknet und unter vermindertem Druck eingeengt. Man erhält auf diese Weise 25,6 g (98% der Theorie) eines Produktes, das gemäß gaschromatischer Analyse zu 93% aus dem Diastereomeren (A) der Formel (I-1) und zu 7% aus dem Diastereomeren (B) besteht. (d. e. 86 %)

Die Diastereomeren können durch Chromatographie an Kieselgel mit Petrolether:Diethylether = 5:1 als Laufmittel getrennt werden. Man erhält dabei das reine Diastereomer (A) der Formel (I-1) in einer Ausbeute von 61 % der Theorie.

Diastereomer (A):

$R_F$-Wert 0,46 (Kieselgel; Petrolether:Diethylether = 5:1)
IR-Spektrum (NaCl) : 3060 $cm^{-1}$ ( = CH )
$^1$H-NMR-Spektrum (360 MHz, $CDCl_3$)
Charakteristische Signale:
$\delta$ = 0,91 (d, 3H, $CH_3$-CH); 0,99 (s, 9H,$^t$Bu-); 1,23 u. 1,43 (je s, je 3H, $CH_3$-C-$CH_3$); 2,82 u. 2,98 (dd, J = 5,2 Hz, 2H,

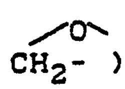 );

3,38 (d von t, 1H,
- CH-OR); 5,48 (s, 1H,
-S- CH-O-)
Massenspektrum:
m/e = 298 (2%, $M^+$)

Diastereomer (B):

$R_F$-Wert 0,29 (Kieselgel; Petrolether:Diethylether = 5:1)
$^1$H-NMR-Spektrum (360 MHz, $CDCl_3$)
Charakteristische Signale:
δ = 0,91 (d, 3H, $C\underline{H}_3$-CH); 1,04 (s, 9H,$^t\underline{B}$u-); 1,24 u. 1,43 (je s, je 3H, $C\underline{H}_3$-C-$C\underline{H}_3$); 2,71 u. 2,93 (dd, J = 4,7 Hz,

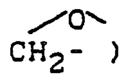 );

3,39 (d von t, 1H, - C$\underline{H}$-OR); 5,30 (s, 1H, - S-CH-O-).
Massenspektrum:
m/e = 298 (2%, $M^+$)

Beispiel 2

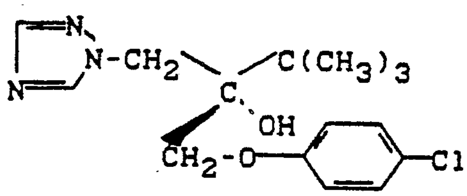

S-Enantiomer (V-1)

1. Stufe

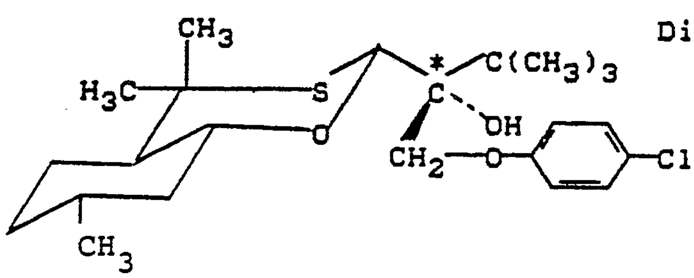

Diastereomer (A)

(VIII-1)

Eine Lösung von 43,1 g (0,34 mol) 4-Chlorphenol in 80 ml absolutem Dimethylformamid wird mit 6,4 g (0,27 mol) Natriumhydrid ( = 8,0 g 80%-iges Natriumhydrid) versetzt und 30 Minuten bei 25 °C gerührt. Danach wird eine Lösung von 25 g (84 mmol) optisch aktivem Oxiran gemäß Beispiel 1 (Rohprodukt) in 50 ml Dimethylformamid zugetropft. Es wird noch 30 Minuten bei 25 °C und eine weitere Stunde bei 120 °C gerührt. Danach wird das Reaktionsgemisch abgesaugt und unter vermindertem Druck eingeengt. Der verbleibende Rückstand wird in ein Gemisch aus Eiswasser und Dichlormethan gegossen. Man trennt die organische Phase ab, extrahiert die wäßrige Phase noch zweimal mit Dichlormethan, wäscht die vereinigten organischen Phasen zweimal mit 10%-iger wäßriger Natronlauge und dann mit wäßriger Kochsalz-Lösung. Nach dem Trocknen über Natriumsulfat wird die organische Phase unter vermindertem Druck eingeengt. Man erhält 32,3 g eines Produktes, das zu 90% aus den beiden Diastereomeren der obigen Formel besteht, wobei in dem Diastereomeren-Gemisch das Diastereomer (A) zu 91,5% und das Diastereomer (B) zu 8,5% enthalten ist (83% d.e.).

Spektroskopische Daten des Diastereomeren-Gemisches:
IR-Spektrum (NaCl): 3600-3300 $cm^{-1}$ (OH);
1600, 1580 $cm^{-1}$ (C=C)
$^1$H-NMR-Spektrum (360 MHz, $CDCl_3$):
Charakteristische Signale:
$\delta$ = 2,83 u. 2,97 (O<u>H</u>); 3,30-3,50 (m, 1H,

- C<u>H</u>-OR);
4,07 bzw. 4,21

(je dd, HO-C-$CH_2$-);

5,23 u. 5,37 (je s,

-S- C<u>H</u>-O-);
6,80-6,97 und 7,22-7,30 (4 H, Arom. H).

Massenspektrum (identisch für beide Diastereomere): m/e 426 ($M^+$ fehlt); 369 (0,5 %, $M^+$ - [t]Bu), 199 (100 %,Oxathian-Fragment).

<u>2. Stufe</u>

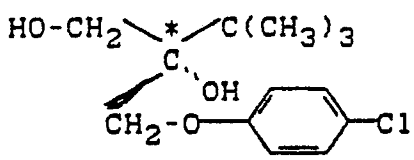

S-Enantiomer
(X-1)

Eine Lösung von 31,9 g (75 mmol) an Rohprodukt des Diastereomeren (A) der Formel (VIII-1) in 250 ml Acetonitril wird unter Rühren in ein auf 40 - 50 °C erwärmtes Gemisch aus 23,9 g (0,18 mol) N-Chlorsuccinimid, 25,4 g (0,15 mol) Silbernitrat, 20,0 g (0,24 mol) Natriumhydrogencarbonat, 800 ml Acetonitril und 150 ml Wasser gegeben. Man rührt noch 15 Minuten bei 40 bis 50 °C, tropft dann nacheinander 78 ml gesättigte, wäßrige Natriumsulfit-Lösung und 78 ml gesättigte, wäßrige Natriumchlorid-Lösung hinzu, saugt ab und wäscht den Rückstand mit einem Gemisch aus Methylenchlorid/Hexan = 1:1.

Das Filtrat wird zweimal mit jeweils dem gleichen Volumen eines Gemisches aus Methylenchlorid/Hexan = 1:1 extrahiert. Die organische Phase wird abgetrennt, mit gesättigter, wäßriger Natriumhydrogencarbonat-Lösung gewaschen, über Natriumsulfat getrocknet, unter vermindertem Druck eingeengt und anschließend im Hochvakuum von noch enthaltenen Lösungsmittel-Resten befreit. Das dabei anfallende Produkte, das zu überwiegendem Anteil aus optisch aktivem $\alpha$-Hydroxyaldehyd der Formel

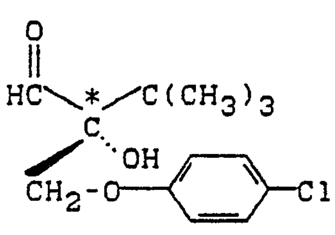

R-Enantiomer
(X-1)

besteht, wird in 250 ml absolutem Diethylether gelöst und bei Raumtemperatur unter Rühren in eine Suspension von 14,1 g (0,37 mmol) Lithium-aluminium-hydrid in 250 ml absolutem Diethylether getropft. Man erhitzt das Reaktionsgemisch 3 Stunden unter Rückfluß, tropft dann nacheinander 25 ml Essigester, 25 ml 4N wäßrige Natronlauge und 25 ml Wasser hinzu, saugt ab, wäscht mit Diethylether nach, trocknet die organische Phase über Natriumsulfat und engt unter vermindertem Druck ein.

Man erhält auf diese Weise 12,9 g eines Produktes, das gemäß gaschromatographischer Analyse zu 60% aus dem optisch aktiven Diol der Formel (X-1) besteht.
IR-Spektrum (NaCl):
OH-Bande bei 3100-3600 $cm^{-1}$.
C=C-Banden bei 1600 und 1590 $cm^{-1}$.
Massenspektrum:
m/e = 259 (20%, $M^+$)

3. Stufe

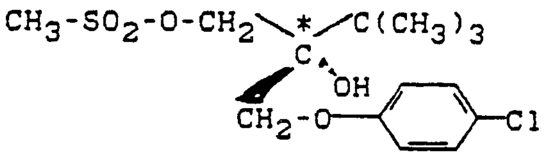

R-Enantiomer
(XII-1)

In eine Lösung von 12,7 g an Rohprodukt des R-Enantiomerem der Formel (X-1) in 20 ml absolutem Methylenchlorid werden unter Stickstoffatmosphäre bei 0 °C unter Rühren gleichzeitig eine Lösung von 5,0 g (44 mMol) Methansulfonsäurechlorid in 10 ml absolutem Methylenchlorid und eine Lösung von 4,4 g (44 mMol) Triethylamin in 10 ml absolutem Methylenchlorid eingetropft. Es wird 4 Stunden bei 0 °C nachgerührt und dann auf 25 °C erwärmt. Man extrahiert das Reaktionsgemisch nacheinander mit gesättigter, wäßriger Citronensäure-Lösung und mit gesättigter, wäßriger Natriumhydrogencarbonat-Lösung, trocknet über Natriumsulfat und engt unter vermindertem Druck ein. Man erhält auf diese Weise 16,9 g an R-Enantiomerem der Formel (XII-1), das ohne zusätzliche Reinigung weiter umgesetzt wird.
IR-Spektrum (NaCl):
OH-Bande 3600-3200 $cm^{-1}$
$CH_3$-$SO_2$-O-$CH_2$-Banden 1360 und 1180 $cm^{-1}$

4. Stufe

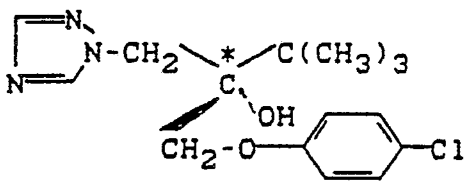

S-Enantiomer
(V-1)

Ein Gemisch aus 16,4 g an Rohprodukt des R-Enantiomeren der Verbindung der Formel (XII-1), 13,3 g Triazol-Natriumsalz und 50 ml absolutem Dimethylformamid wird unter Stickstoffatmosphäre 4 Stunden bei 120 °C gerührt. Danach wird unter vermindertem Druck eingeengt und der verbleibende Rückstand in ein Gemisch aus Wasser und Methylenchlorid gegeben. Man trennt die organische Phase ab, extrahiert die wäßrige Phase zweimal mit Methylenchlorid, trocknet die vereinigten organischen Phasen über Natriumsulfat und engt ein. Es verbleiben 9,04 g eines Produktes, das durch Säulenchromatographie an 350 g Kieselgel mit einem Gemisch aus Methylenchlorid/Methanol = 95:5 als Laufmittel gereinigt wird. Durch Einengen des Eluates erhält man 4,43 g (29 % der Theorie) eines Produktes, das gemäß flüssigkeitschromatographischer Analyse zu 93,5 % aus dem S-Enantiomeren des 2-(4-Chlorphenoxymethyl)-3,3-dimethyl-1-(1,2,4-triazol-1-yl-methyl)-butan-2-ols besteht.
(Optische Reinheit 87% e.e.)

**Ansprüche**

1. Optische aktive Oxirane der Formel

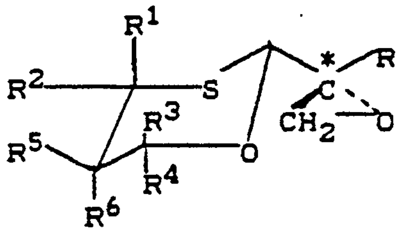

(I)

in welcher
R für gegebenenfalls substituiertes Alkyl, gegebenenfalls substituiertes Cycloalkyl oder für gegebenenfalls substituiertes Phenyl steht und
$R^1$, $R^2$, $R^3$, $R^4$, $R^5$ und $R^6$ für Wasserstoff oder Alkyl stehen, wobei jedoch mindestens einer der Reste für Alkyl steht, oder
$R^4$ und $R^5$ gemeinsam für gegebenenfalls durch Alkyl substituiertes Alkandiyl stehen oder
$R^4$ und $R^5$ gemeinsam mit den angrenzenden Kohlenstoffatomen für einen gegebenenfalls durch Alkyl substituierten, anellierten bicyclischen Kohlenwasserstoff-Rest stehen.

2. Optisch aktive Oxirane der Formel (I) gemäß Anspruch 1, in denen
R für gegebenenfalls durch Halogen, Alkoxy mit 1 bis 4 Kohlenstoffatomen, Alkylthio mit 1 bis 4 Kohlenstoffatomen, die CHO-Gruppe und deren Derivate, Phenoxy und/oder Phenyl substituiertes geradkettiges oder verzweigtes Alkyl mit 1 bis 6 Kohlenstoffatomen, für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen und/oder Halogen substituiertes Cycloalkyl mit 3 bis 8 Kohlenstoffatomen oder für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, und/oder Halogen substituiertes Phenyl steht und
$R^1$, $R^2$, $R^3$, $R^4$, $R^5$ und $R^6$ für Wasserstoff oder Alkyl mit 1 bis 4 Kohlenstoffatomen stehen, wobei jedoch mindestens einer dieser Reste für Alkyl mit 1 bis 4 Kohlenstoffatomen steht, und außerdem
$R^4$ und $R^5$ auch gemeinsam für gegebenenfalls ein-bis dreifach, gleichartig oder verschieden durch Alkyl mit 1 bis 4 Kohlenstoffatomen substituiertes Alkandiyl mit 3 bis 6 Kohlenstoffatomen stehen oder auch
$R^4$ und $R^5$ gemeinsam mit den angrenzenden Kohlenstoffatomen für einen gegebenenfalls ein- bis dreifach, gleichartig oder verschieden durch Alkyl mit 1 bis 4 Kohlenstoffatomen substituierten, annellierten bicyclischen Kohlenwasserstoff mit 7 oder 8 Kohlenstoffatomen stehen.

3. Verfahren zur Herstellung von optisch aktiven Oxiranen der Formel

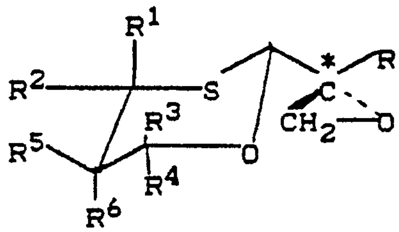

(I)

in welcher
R für gegebenenfalls substituiertes Alkyl, gegebenenfalls substituiertes Cycloalkyl oder für gegebenenfalls substituiertes Phenyl steht und
$R^1$, $R^2$, $R^3$, $R^4$, $R^5$ und $R^6$ für Wasserstoff oder Alkyl stehen, wobei jedoch mindestens einer der Reste für Alkyl steht, oder
$R^4$ und $R^5$ gemeinsam für gegebenenfalls durch Alkyl substituiertes Alkandiyl stehen oder
$R^4$ und $R^5$ gemeinsam mit den angrenzenden Kohlenatomen für einen gegebenenfalls durch Alkyl substituierten, anellierten bicyclischen Kohlenwasserstoff-Rest stehen,
dadurch gekennzeichnet, daß man enantiomeren-reine Oxathian-Ketone der Formel

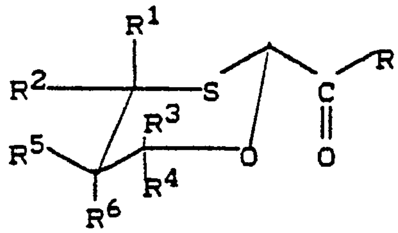

(II)

in welcher
R, $R^1$, $R^2$, $R^3$, $R^4$, $R^5$ und $R^6$ die oben angegebene Bedeutung haben,
entweder α) mit Sulfonium-Salzen der Formel

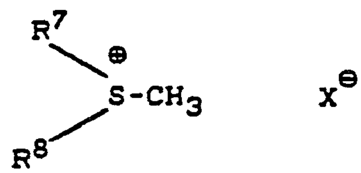

(III)

in welcher
$R^7$ für Methyl oder Phenyl steht,
$R^8$ für Methyl oder Phenyl steht und
$X^{\ominus}$ für Halogenid oder Methosulfat steht,
oder
β) mit Sulfoxonium-Salzen der Formel

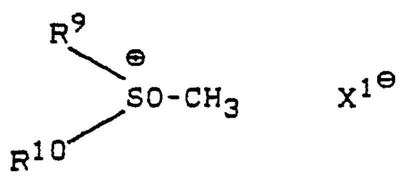

(IV)

in welcher
$R^9$ für Methyl oder Phenyl steht,
$R^{10}$ für Methyl oder Phenyl steht und
$X^{1\ominus}$ für Halogenid oder Methosulfat steht,
in Gegenwart einer starken Base und in Gegenwart eines Verdünnungsmittels bei Temperaturen zwischen -78° C und +100° C umsetzt.

4. Verfahren zur Herstellung von optisch aktiven 2-Hydroxyethyl-azol-Derivaten der Formel

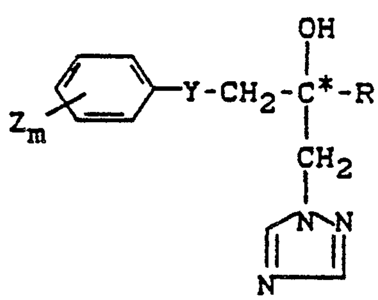

(V)

in welcher
R für gegebenenfalls substituiertes Alkyl, gegebenenfalls substituiertes Cycloalkyl oder für gegebenenfalls substituiertes Phenyl steht,
Y für Sauerstoff, eine direkte Bindung oder für eine $CH_2$-Gruppe steht,

Z für Halogen, Alkyl, Cycloalkyl, Alkoxy, Alkylthio, Halogenalkyl, Halogenalkoxy, Halogenalkylthio, gegebenenfalls substituiertes Phenoxy, gegebenenfalls substituiertes Phenylalkyl oder gegebenenfalls substituiertes Phenoxyalkyl steht und

m für die Zahlen 0, 1, 2 oder 3 steht,

dadurch gekennzeichnet, daß man optisch aktive Oxirane der Formel

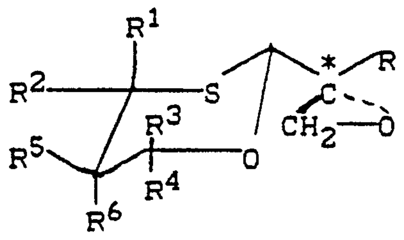

(I)

in welcher

R, $R^1$, $R^2$, $R^3$, $R^4$, $R^5$ und $R^6$ die oben angegebene Bedeutung haben,

in einer ersten Stufe entweder

γ) mit Phenol-Derivaten der Formel

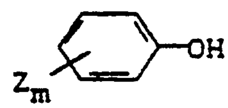

(VI)

in welcher

Z und m die oben angegebene Bedeutung haben,

in Gegenwart einer Base und in Gegenwart eines Verdünnungsmittel bei Temperaturen zwischen 0° C und 150° C umsetzt,

oder

δ) mit Grignard-Verbindungen der Formel

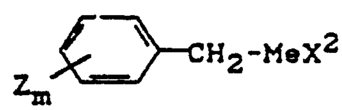

(VII)

in welcher

Z und m die oben angegebene Bedeutung haben,

Me für ein Erdalkalimetall oder für Zink steht, und

$X^2$ für Chlor, Brom oder lod steht,

in Gegenwart eines Verdünnungsmittels bei Temperaturen zwischen -78° C und +100° C umsetzt,

danach in einer zweiten Stufe die so erhaltenen optisch aktiven Verbindungen der Formel

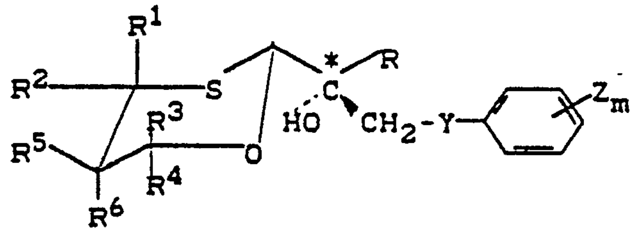

(VIII)

in welcher

R, $R^1$, $R^2$, $R^3$, $R^4$, $R^5$, $R^6$, Y, Z und m die oben angegebene Bedeutung haben,

mit einem zur Spaltung von Oxathian-Verbindungen geeigneten Reagenz in Gegenwart eines Verdünnungsmittels sowie gegebenenfalls in Gegenwart eines Säurebindemittels bei Temperaturen zwischen 0° C und 100° C umsetzt und die dabei entstehenden optisch aktiven α-Hydroxyaldehyde der Formel

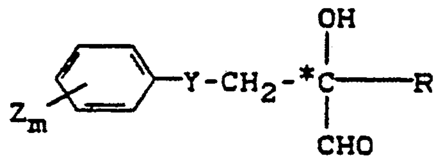

(IX)

in welcher
R, Y, Z und m die oben angegebene Bedeutung haben,
mit einem zur Reduktion von Aldehyden geeigneten Reagenz in Gegenwart eines Verdünnungsmittels bei Temperaturen zwischen -20 °C und +100 °C umsetzt,
weiterhin in einer dritten Stufe die so erhaltenen optisch aktiven Diole der Formel

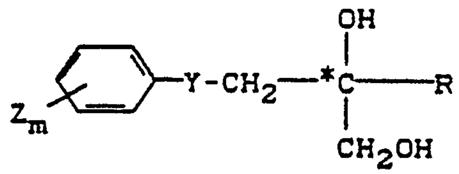

(X)

in welcher
R, Y, Z und m die oben angegebene Bedeutung haben,
mit Sulfonsäure-Derivaten der Formel
$R^{11}$-$SO_2$-Hal (XI)
in welcher
$R^{11}$ für Alkyl, Halogenalkyl oder gegebenenfalls substituiertes Phenyl steht und
Hal für Halogen steht,
in Gegenwart eines Verdünnungsmittels und in Gegenwart eines Säurebindemittels bei Temperaturen zwischen 0 °C und 100 °C umsetzt und schließlich in einer vierten Stufe die entstandenen optisch aktiven Verbindungen der Formel

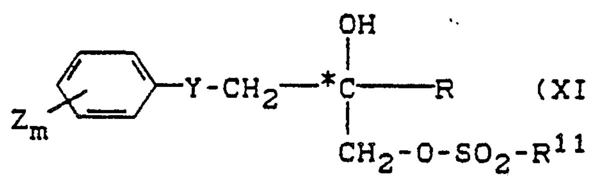

(XII)

in welcher
R, $R^{11}$, Y, Z und m die oben angegebene Bedeutung haben,
mit Triazol-Salzen der Formel

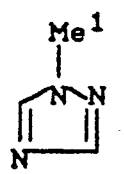

(XIII)

in welcher
$Me^1$ für ein Alkalimetall steht,
in Gegenwart eines Verdünnungsmittels und gegebenenfalls in Gegenwart eines Säurebindemittels bei Temperaturen zwischen 20 °C und 150 °C umsetzt.